# EUROPEAN PATENT APPLICATION

(11) **EP 0 891 751 A1**
(43) Date of publication of application: **20.01.1999**
(21) Application number: 97112382.3
(22) Date of filing: 18.07.1997
(51) Int. Cl.: A61F 2/06

(54) **Vascular stent for bifurcations, sidebranches and ostial lesions and an application catheter and method for implantation**

(71) Applicant: Ischinger, Thomas, Prof. Dr., 81927 München (DE)
(72) Inventor: Ischinger, Thomas, Prof. Dr., 81927 München (DE)
(74) Representative: Ruschke, Hans Edvard, Dipl.-Ing.

(57) **Abstract**

A dilatation catheter with a most distal guidewire exit located distal to the dilatation means portion and at least one more proximally located wire exit, said wire exit being located within the portion of the dilatation means.

The application also relates to a self-expandable stent whose at least one end is in an oblique plane to the longitudinal axis of the stent and to a positioning wire whose distal tip has a pigtail configuration.

## Description

Vascular stent for bifurcations, sidebranches and ostial lesions and an application catheter and method for implantation.

### Background:

Stents are prostheses to support the lumen of hollow organs, primarily to acutely maintain the lumen of blood vessels after mechanical interventions such as balloon angioplasty and to achieve a better long term result after such mechanical interventions. While implantation of stents into straight vessel segments poses little technical problems, implantation of stents into ostial lesions , sidebranches or into vessel bifurcations represents a challenge to the operator and carries increased risks of acute and longterm failure, in particular due to misplacement or imprecise placement.

In ostial lesions, the proximal end of the stent must be precisely placed at the ostium of the artery so that the stent is not protruding into the aortic lumen. Also, in order to avoid the above risk, the stent may be advanced to far into the artery so that the initial segment of the diseased ostium remains unstented.

A similar problem exists with stenting of sidebranches and vessel bifurcations. For both situations, precision placement techniques are required for optimal results. However, the operator must rely on visual assessment during fluoroscopy with and without contrast injections. Contrast injections are of little help for stenting in ostial lesions, since opacification of the target artery is usually inadequate and identification of the aortic lumen and the ostial takeoff is very limited. In sidebranch and bifurcational lesions, precise placement is similarly difficult due to poor identification of the exact beginning of the sidebranch ostium and the often nonperpendicular nature of the plane of the sidebranch in relation to the axis of the major vessel. The beating heart makes maintaining of a catheter position with current techniques even more difficult if not impossible.

The current invention offers a unique solution to the technical problems as described above.

### Description of the device and method:

A balloon catheter as used for vascular dilatation commonly has a balloon inflation channel along the whole length of the shaft of the balloon catheter up to the beginning or the midportion of the balloon, and a so called wire channel through which the angioplasty guidewire is being fed. The guidewire channel commonly exits at the distal end of the balloon catheter (very distal catheter tip). Depending on whether the guidewire channel is running through the whole length of the balloon catheter shaft and exiting proximally at the proximal end of the balloon catheter shaft or whether the guidewire channel is exiting via a side-exit just proximal to the balloon, the balloon catheters are termed over the wire or monorail balloon catheters.

In order to create a technique to assist to mechanically self-position a balloon in an ostial, sidebranch or bifurcational lesion, the balloon must have a mechanical means to limit its advancement beyond a certain point. This is realized by an additional wire exit located within the inflatable portion of the balloon catheter used for stenosis dilatation (within the distal and proximal end of the balloon catheter) or within the respective portion used for stenosis dilatation or stent application of any other mechanical dilatation catheter or located within a portion of 6 mm length beginning just proximal to the functional segment of an application catheter for selfexpanding stents or application of other therapeutics and extending proximally.Such wire exit within the ballon segemnt or as described above will be termed "extra distal wire exit" in any further description.
The extra distal wire exit is the distal end of a wire channel that may run through the whole length of the balloon catheter shaft and exit proximally (over the wire fashion) or the distal end of a wire channel that is shorter than the shaft of the catheter and therefore exit from the shaft at any location proximal to the balloon segment or the functional segment as described above. Preferentially, the the proximal exit of the extra wire channel is within the the distal half of the catheter so that monorail handling of such extra wire channel and extra wire is possible.

It may be a preferred embodiment, that the extra wire channel merges with the over the wire guidewire channel over a certain distance until the extra wire exits sideways or that the extra wire channel merges with the monorail guidewire channel and use the same proximal side exit as the monorail guidewire channel.

Technique of use: If a monorail type balloon catheter is used for bifurcational (or sidebranch) dilatation and stenting, first a guidewire is placed in the target artery(sidebranch); then the extra wire is placed in the main artery. Then the balloon catheter is advanced over the guide wire ( placed in target lesion) which is threaded through the distal opening of the guidewire channel at the balloon catheter tip *and* over the extra wire (main artery), which is threaded through the extra distal wire exit. The balloon is then advanced until the extra distal wire exit is reaching the bifurcation and prohibits any further advancement. one or more radioopaque markers make(s) the location of the extra distal wire exit visible to the operator.
This procedure permits stable positioning of a balloon catheter within a bifurcation. But, more importantly, it orients the extra distal wire exit automatically into the direction of theopening to the main artery, prohibiting any further rotation of the balloon, which may carry a stent.Thereby, stents with oblique ends may be placed into sidebranches.

For stable balloon placement in ostial lesions, prepositioning of the extra wire is not necessary and not easily achievable. In these cases, the extra distal wire channel may be preloaded and the extra wire may be advance through the extra distal wire exit once the balloon is approachin the target ostium. For ostial lesions, more than one extra distal wire channel may be helpful and the wires used need no steerability but rather a atraumatic distal configuration and floppy property, e.g. mini-pigtail shape, superelasticity (e.g.nitinol).

The bifurcational or sidebranch stent:
the longitudinal axis of sidebranches are almost never perpendicular to the longitudinal axis of the vessel from which they are taking off. Therefore current stent configurations, which are commonly of cylindrical tubular shape with the crossectional plane of the ends of the stent being perpendicular to the longitudinal axis of the cylinder (stent). This configuration does not permit full stent coverage of the ostium of a sidebrance, as there is alwasy the risk of protrusion of one edge of the end of the stent into the lumen of the main artery. Stents with at least one oblique (oblong?) end are needed to better adapt to bifurcational anatomies, in particular the vessel takeoff angles.

A stent is described, where one end of the stent cylinder is cut in a plane non perpendicular to the longitudinal axis of the stent, i.e. oblique, preferentially in an angle of 80 to 45 degrees to the longitudinal axis of the stent (or 10 to 45 degrees to the axis perpendicular to the longitudinal axis of the stent)

This configuration i sdefined by a maximum and a minimum length of a stent, where the Mantellinie of maximum length opposing the Mantellinie of minimum length at 180°.

Placement of such stent and use of the potential of such stent is only possible, if the stent carrying instrument,e.g.balloon catheter, can be directed so that the long and short sides of the stent are placed correctly in the ostium. This can be achieved by the selforienting catheter with an extra distal wire exit. The bifurcational stent will be mounted on the balloon in such a manner, that the shortest portion of the oblique end is closest to the extra distal wire exit.

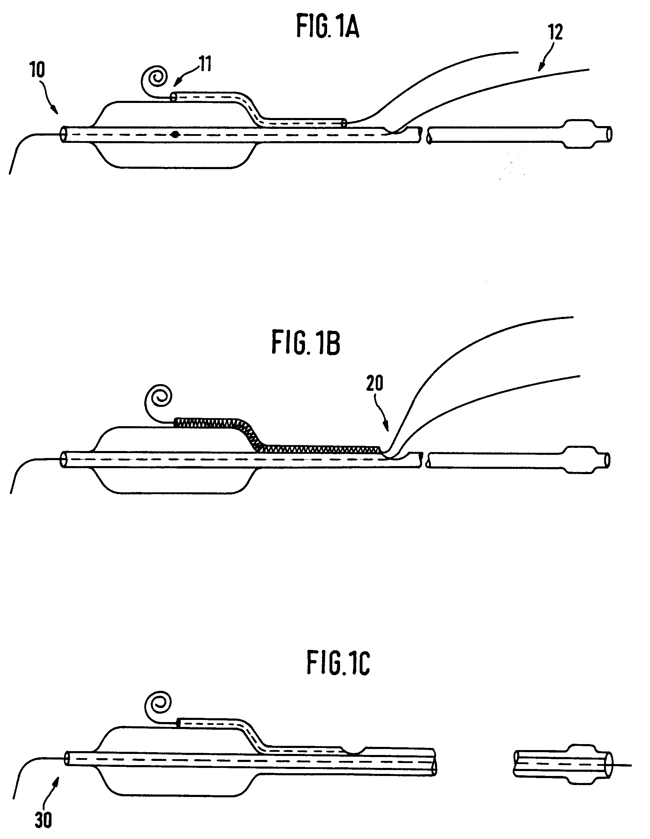

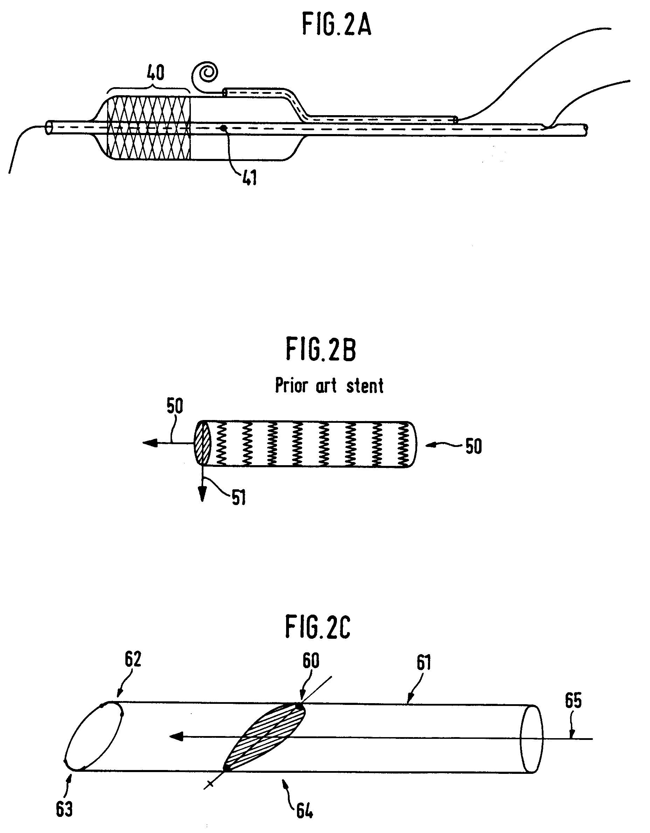

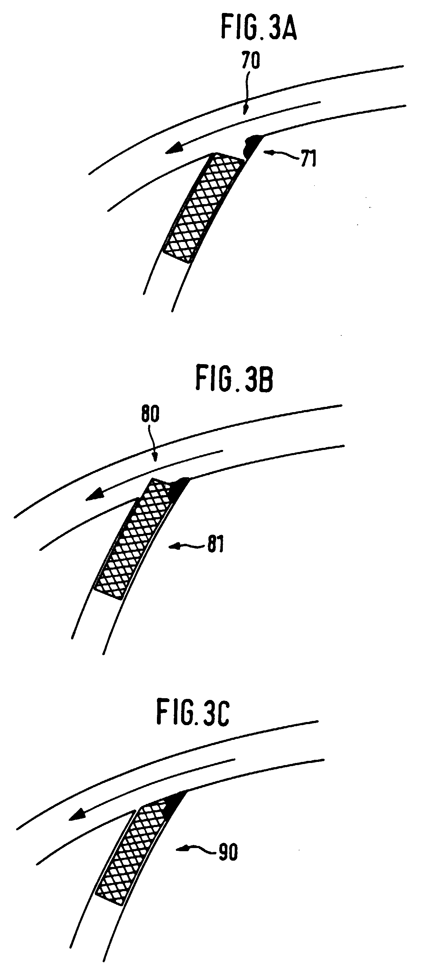

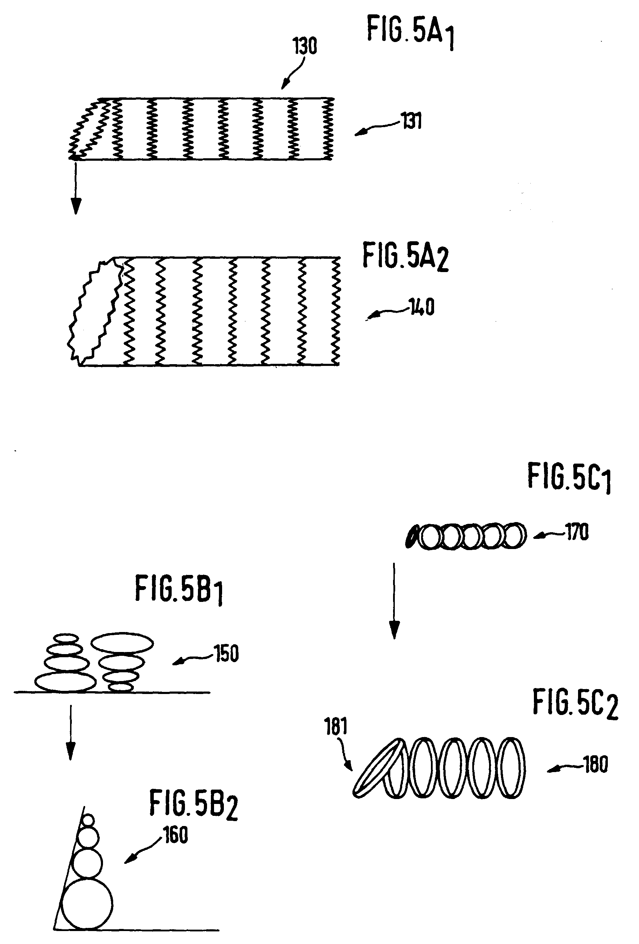

This description is an example and does not include by far any conceivable elements and techniques incorporated and related to this technique and this novel balloon and novel stent.
Figures:
   **Sheet 1:**
   Figures: "Bifurcational Balloon and Stent Technique "
Fig. 1 A: Balloon or other dilatation means of claim 1
   - 10: distal tip
   - 11: more proximally located wire exit for positioning wire
   - 12: regular guide wire (monorail)
Fig. 1 B
   - 20: both wire channels "merge" and use one proximal exit - arrow
Fig. 1 C
   - 30: guidewire has "through" lumen and exits at proximal end of shaft (non- monorail balloon catheter)
   **Sheet 2:**
   Stent of Claim 1 on Balloon
Fig. 2A
   - 40: stent
   - 41: radioopaque marker
Fig. 2 B:
   Oblique stent (common stent)
   - 50: longitudinal axis of stent
   - 51: axis of crossectional plane of stent end = (perpendicular to longitudinal axis of stent)
Fig. 2 C:
   Novel stent for side branches or bifurcation
   - 60: axis non-perpendicular to longitudinal axis of stent
   - 61: minimum "Mantellinie"
   - 62: opposing ends of minimum
   - 63: maximum length "Mantellinie"
   - 64: maximum "Mantellinie"
   - 65: longitudinal axis of stent
   **Sheet 3:**
   Stent and Balloon in artery
Fig. 3A:
   Normal stent
   - 70: main artery
   - 71: lesion uncovered by stent
Fig. 3B
   - 80: edge of one end protruding into main artery
   - 81: lesion partially covered by stent
Fig. 3C:
   Novel stent
   - 90: lesion covered and no protrusion of stent
   **Sheet 4:**

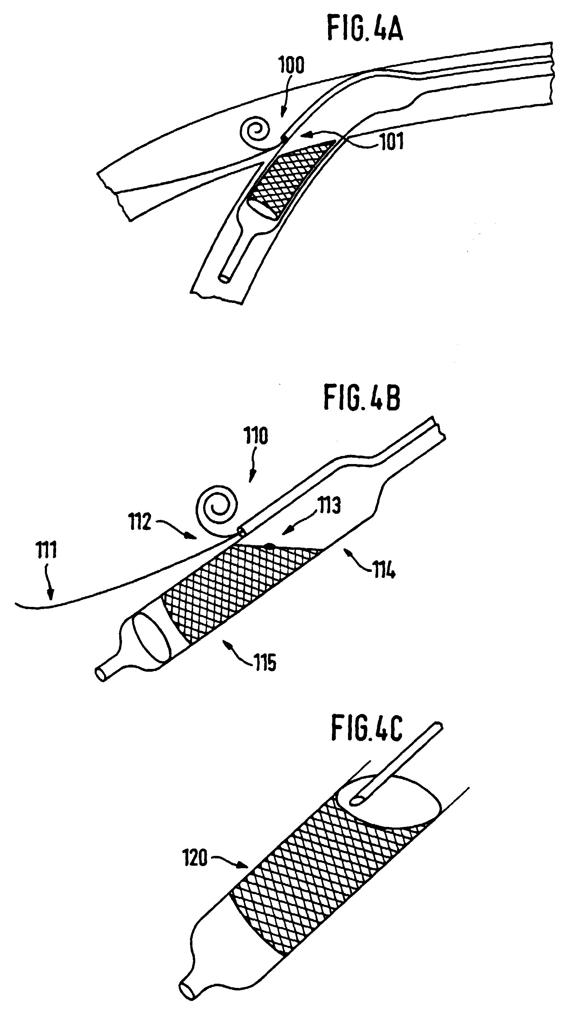

   Figures 4 - Precision placement of novel stent on novel balloon catheters (dilatation catheter)
Fig. 4A
   - 100: extra wire exit
   - 101: extra (more proximally located) wire exit facilitates positioning of balloon across bifurcation; stent is mounted on distal half of balloon (distal to extra wire exit)
Fig. 4B
   - 110: pigtail wire
   - 111: or normal wire
   - 112: short "Mantellinie" of stent
   - 113: marker(s) on shaft or balloon
   - 114: long "Mantellinie"
   - 115: oblique stent
Fig. 4C
   - 120: crossection
   Sheet 5:
   Stent configurations
Fig. 5A₁
   - 130: sinusoidal rings (zig zag)
   - 131: stretched angles of end-segment non-expanded
Fig. 5A₂
   - 140: stretched angles of end-segment expanded
Fig. 5B₁
   - 150: closed loops non-expanded
Fig. 5B₂
   - 160: closed loops expanded
Fig. 5C₁
   - 170: ratcheting bands
Fig. 5C₂
   - 180: oblique end-ratcheting band
   - 181: expanded

## Claims

1. A dilatation catheter (balloon or other dilatation means) with a most distal guidewire exit located distal to the inflatable balloon portion or other dilatation means and at least one more proximally located wire exit, said wire exit located *within* the inflatable portion of the balloon or other fuctional portion of any other dilatation means (*within the distal and proximal end of the inflatable balloon or other functional dilatation means)*.

2. A tubular balloon or selfexpandable stent with the crossectional plane of at least one end being nonperpendicular to the longitudinal axis of the tubular stent, so that there is a Mantellinie of maximum length and one Mantellinie of minimum length which are opposing or nearly opposing each other within 160 to 200°.

3. A dilatation catheter of claim one in combination with a stent of claim 2 used as bare or premounted stent, wherein the stent is mounted on the portion of the balloon or other dilatation means which is distal to the more proximally located wire exit of claim 1, and wherein said stent of claim 2 is oriented on the balloon or other dilatation means in such a manner, that the extension of the longitudinal axis of the Mantellinie of minimum length is pointing to the more proximally located wire exit of claim 1 (*or the continuation of the axis of the Mantellinie with minimum length is crossing the more proximally located wire exit of claim 1 oder is on the same Mantellinie as the more peoximally located wire exit of claim 1 oder so ähnlich*).

4. Dilatation catheter of claim 1, wherein the at least one more proximally located wire exit within the inflatable portion of the balloon is the distal exit of a wire channel (*fluid connection with a wire channel*), said wire channel having its proximal exit from the catheter shaft through the proximal end or a near proximal second end of the cathetershaft.

5. Dilatation catheter of claim 1, wherein the at least one more proximally located wire exit within the inflatable portion of the balloon is the distal exit of a wire channel, said wire channel having its proximal exit from the shaft through a sidehole at any selected distance from the proximal end of the inflatable portion of the balloon or the functional portion of any othet dilatation means.

6. Dilatation catheter of claim 4 or 5, wherein said wire channel being in fluid connection with the more proximally located wire exit within the inflatable portion of the balloon / dilatation means is merging over a distance (*or: is in fluid connection*) with the regular guide wire channel, the distal exit of which is distal to the inflatable portion of the balloon/dilatation means.

7. A tubular stent of claim 2, said stent having at least one oblique end, wherein in the expanded state the oblique end is formed by a circumferentially extending ring like band, said band having an axis of its crossectional plane that is non perpendicular to the longitudinal axis of the tubular stent.

8. Stent of claim 7, wherein the oblique end is formed by a sereis of closed loop elements aligned circumferentially to form a tubular segement, wherein at least two such such loops have different lenghts of their longitudinal axis.

9. Stent of claim 7, wherein the oblique end is formed by sinusoidally bent (zig zig) wire like material extending circumferentially, both ends of said sinusoidal structure being closed around a thought cylinder to form a tubular segment, whrein the crossectional plane of such tubular segment is non perpendicular to the longitudinal axis of the stent, and wherein the the sinusoidal configuration is being transformed upon expansion of the tubular segment into a essentioally ring like structure, wherein all angles in the circumferential axis of the formerly sinusoidal structure are between 140°and 180°.

10. A positioning wire for use in combination with balloon of claim 1, wherein the distal tip of such wire is having a pigtail configuration of at least 1 full 360° loop, and said wire having a distal segment of at least 5 cm of superelastic property (eg Nitinol) and radioopacity.

11. Catheter of claim 1, wherein the more proximally located wire exit within the inflatable portion of the balloon / functional portion of aothet dilatation means is marked by at least one radioopaque marker element on the balloon or the dilatation means or on the catheter shaft.
